# EUROPÄISCHE PATENTSCHRIFT

(11) **EP 0 612 723 B1**
(45) Veröffentlichungstag und Bekanntmachung des Hinweises auf die Patenterteilung: **27.08.1997**
(21) Anmeldenummer: 94102322.8
(22) Anmeldetag: 16.02.1994
(51) Int. Cl.: C07C 279/22, A61K 31/155

(54) **Substituierte Benzoylguanidine, Verfahren zu ihrer Herstellung, ihre Verwendung als Medikament, als Inhibitoren des zellulären Na+/H+-Austauschs oder als Diagnostikum sowie sie enthaltendes Medikament**
Substituted benzoylguanidines, process for their preparation, their use as medicament, as inhibitor of the cellular Na+/H+ exchange or as diagnostic agent and medicament containing them
Benzoylguanidines substituées, procédé de leur préparation, leur utilisation comme médicament, comme inhibiteurs de l'échange cellulaire de Na+/H+ ou comme agent diagnostique et médicament les contenant

(30) Priorität: 20.02.1993 DE 4305250
(43) Veröffentlichungstag der Anmeldung: 31.08.1994
(73) Patentinhaber: HOECHST AKTIENGESELLSCHAFT, 65929 Frankfurt am Main (DE)
(72) Erfinder: Lang, Hans-Jochen, Dr., D-65719 Hofheim/Taunus (DE); Weichert, Andreas, Dr., D-60529 Frankfurt/Main (DE); Kleemann, Heinz-Werner, Dr., D-61350 Bad Homburg (DE); Schwark, Jan-Robert, Dr., D-65929 Frankfurt/Main (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- EP-A- 0 556 672
- EP-A- 0 556 674
- WO-A-84/00875
- DE-A- 3 502 629
- US-A- 4 251 545

## Beschreibung

Die Erfindung betrifft Benzoylguanidine der Formel I 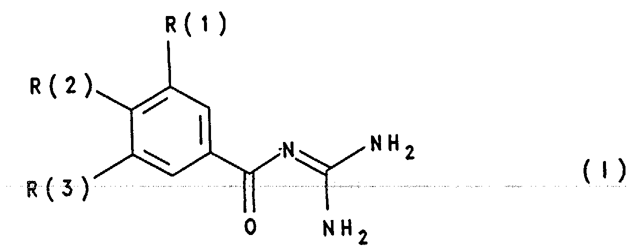 worin bedeuten:
R(1), R(2), R(3)
   Wasserstoff, F, Cl, Br, I, (C₁-C₁₂)-Alkyl,
einer der Substituenten R(1), R(2) oder R(3)
   N₃, CN, OH oder (C₁-C₁₀)-Alkyloxy, wenn mindestens einer der restlichen Substituenten R(1), R(2) oder R(3) einen hinreichend lipophilen Alkylrest mit 3 bis 12 Kohlenstoff-Atomen bedeutet,
oder einer der Substituenten R(1), R(2) oder R(3) ist
R(4)-CₙH₂ₙ-Oₘ- mit
m gleich Null oder 1,
n gleich Null, 1, 2 oder 3,
R(4) gleich CₚF₂ₚ₊₁
mit p gleich 1, 2 oder 3, sofern n gleich Null oder 1 ist, oder
R(4) gleich (C₃-C₁₂)-Cycloalkyl, oder Phenyl, wobei Phenyl unsubstituiert ist oder substituiert mit einem Substituenten aus der Gruppe
F, Cl, CF₃, Methyl, Methoxy, NR(5)R(6), mit R(5) und R(6) gleich Wasserstoff oder (C₁-C₄)-Alkyl
oder einer der Substituenten R(1), R(2) oder R(3)
-C≡CR(5), -C[R(6)] = CR(5),
R(5) Phenyl,
das unsubstituiert oder substituiert ist mit 1 - 3 Substituenten aus der Gruppe F, Cl, CF₃, Methyl, Methoxy, Hydroxy, Amino, Methylamino oder Dimethylamino,
(C₁-C₉)-Heteroaryl,
   das unsubstituiert oder wie Phenyl substituiert ist,
(C₁-C₆)-Alkyl,
   das unsubstituiert oder mit 1 - 3 OH substituiert ist, oder
(C₃-C₈)-Cycloalkyl,
R(6) Wasserstoff, Methyl,
sowie deren pharmakologisch akzeptable Salze,
mit Ausnahme der Verbindungen Benzoyl-guanidin, 4-Chlorbenzoyl-guanidin, 3,4-Dichlorbenzoyl-guanidin, 3- oder 4-Methylbenzoyl-guanidin.

Bevorzugt sind Verbindungen, in denen bedeuten:
R(1), R(2), R(3)
Wasserstoff, F, Cl, Br, (C₁-C₈)-Alkyl,
einer der Substituenten R(1), R(2) oder R(3)
OH oder (C₁-C₆)-Alkyloxy, wenn mindestens einer der restlichen Substituenten R(1), R(2) oder R(3) einen hinreichend lipophilen Alkylrest mit 3 bis 6 Kohlenstoff-Atomen bedeutet,
oder einer der Substituenten R(1), R(2) oder R(3) ist
R(4)-CₙH₂ₙ-Oₘ- mit
m gleich Null oder 1,
n gleich Null, 1, 2 oder 3,
R(4) gleich CₚF₂ₚ₊₁
   mit p gleich 1, sofern n gleich Null oder 1 ist, oder
R(4) gleich (C₅-C₇)-Cycloalkyl, oder Phenyl, wobei Phenyl unsubstituiert ist oder substituiert mit einem Substituenten aus der Gruppe
   F, Cl, CF₃, Methyl, Methoxy,
oder einer der Substituenten R(1), R(2) oder R(3)
-C≡CR(5),
R(5) Phenyl, (C₁-C₄)-Alkyl,
das unsubstituiert oder mit OH substituiert ist,
sowie deren pharmakologisch akzeptable Salze,
mit Ausnahme der Verbindungen Benzoyl-guanidin, 4-Chlorbenzoyl-guanidin, 3,4-Dichlorbenzoyl-guanidin, 3- oder 4-Methylbenzoyl-guanidin.

Besonders bevorzugt sind 3-Trifluormethylbenzoyl-guanidin-hydrochlorid, 3,5-Bis-trifluormethylbenzoyl-guanidin-hydrochlorid, 3-Methyl-5-trifluorm ethylbenzoylguanidin-hydrochlorid, 4-Fluor-3-trifluormethyl-benzoylguanidin-hydrochlorid, 4-(4-Fluor-phenoxy)-3-trifluormethyl-benzoylguanidin-hydrochlorid, 5-Fluor-3-trifluormethyl-benzoylguanidin-hydrochlorid, 3-Chlor-4-isopropylbenzoyl-guanidin-hydrochlorid, 4-tert.Butyl-3-methoxybenzoyl-guanidin-hydrochlorid, 3-tert.Butyl-4-hydroxybenzoyl-guanidin-hydrochlorid, 3-tert.Butyl-4-isopropylbenzoyl-guanidin-hydrochlorid, sowie deren pharmakologisch akzeptable Salze.

Enthält einer der Substituenten R(1) bis R(3) ein Asymmetriezentrum, so gehören sowohl S als auch R konfigurierte Verbindungen zur Erfindung. Die Verbindungen können als optische Isomere, als Diastereomere, als Racemate oder als Gemische derselben vorliegen.

Die bezeichneten Alkylreste können sowohl geradkettig wie verzweigt vorliegen.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Die Erfindung betrifft weiterhin ein Verfahren zur Herstellung einer Verbindung I, das dadurch gekennzeichnet ist, daß man
eine Verbindung der Formel II 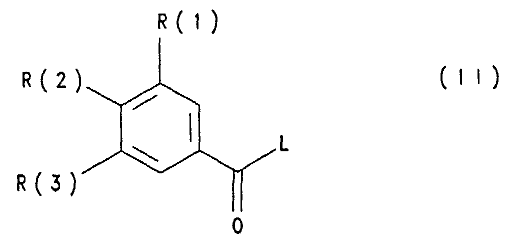 mit Guanidin umsetzt, worin R(1) bis R(4) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L=Cl), die man ihrerseits wiederum in an sich bekannter Weise aus den zugrundeliegenden Carbonsäuren (Formel II, L= OH) beispielsweise mit Thionylchlorid herstellen kann.
Neben den Carbonsäurechloriden der Formel II (L=Cl) lassen sich auch weitere aktivierte Säurederivate der Formel II in an sich bekannter Weise direkt aus den zugrundeliegenden Benzoesäure-Derivaten (Formel II, L = OH) herstellen, wie beispielsweise die Methylester der Formel II mit L=OCH₃ durch Behandeln mit gasförmigem HCl in Methanol, die Imidazolide der Formel II durch Behandeln mit Carbonyldiimidazol (L= 1-Imidazolyl, Staab, Angew. Chem. Int. Ed. Engl. 1, 351-367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluo borat ("TOTU")(Proceedings of the 21. European Peptide Symposium, Peptides 1990, Editors E. Giralt und D. Andreeu, Escom, Ledien, 1991). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L=OMe) mit Guanidin Methanol oder THF zwischen 20°C und Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II und III verwendet werden.

Wenn L=Cl bedeutet, arbeitet man vorteilhaft unter Zusatz eines Säurefängers, z.B. in Form von überschüssigen Guanidin zur Abbindung der Halogenwasserstoffsäure.

Ein Teil der zugrundeliegenden Benzoesäure-Derivate der Formel II sind bekannt und in der Literatur beschrieben. Die unbekannten Verbindungen der Formel II können nach literaturbekannten Methoden hergestellt werden.

Carbonsäuren oder deren Ester der Formel II (z.B. L = -OH oder -O-Methyl) mit R(2) in der Bedeutung von Halogen oder R(3) in der Bedeutung von Nitro können als vielseitige Ausgangsverbindungen für weitere Carbonsäuren der Formel II dienen, wobei das Halogen in der Position R(2) sehr bequem in bekannter Weise gegen zahlreiche nucleophile Reagenzien, wie Phenole oder Alkohole R(4)-CₙH₂ₙ-OH bzw. deren Alkalisalze unter Bildung entsprechender Benzoesäure-Derivate ausgetauscht werden. Ebenso können Nitrogruppen nach Reduktion zur entsprechenden Aminobenzoesäure durch Sandmeier- oder Ullmann-Reaktionen zu gewünschten, insbesondere halogen-substituierten Benzoesäure-Derivaten führen. Chlor, Brom oder lod kann in vielen Fällen auch durch direkte Halogenierung mit Hilfe eines Friedel-Crafts-Katalysators in an sich bekannter Weise in eine jeweilige Benzoesäure eingeführt werden.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 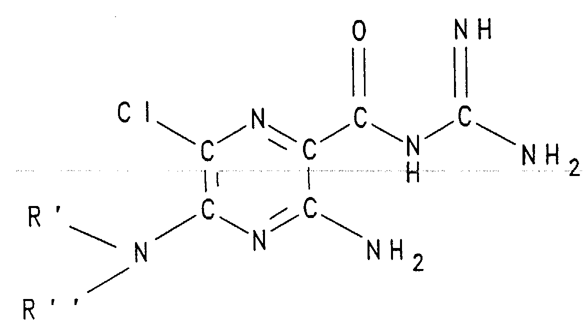 Amilorid: R',R"= H
Dimethylamilorid: R',R''= CH₃
Ethylisopropylamilorid: R'= C₂H₅, R'' = CH(CH₃)₂

Darüberhinaus sind Untersuchungen bekannt geworden, die auf antiarrhythmische Eigenschaften von Amilorid hinweisen (Circulation 79, 1257-63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.
Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der europäischen Offenlegungsschrift 416 499 (HOE 89/F 288) sind Benzoylguanidine beschrieben, die in der dem Rest R(1) entsprechenden Stellung ein Wasserstoffatom tragen.

Aus der deutschen Offenlegungsschrift 3 502 629 sind in m-Stellung mit einer Phenoxy-Gruppe substituierte Benzoylguanidine bekannt, die in der Phenoxy-Gruppe stets mindestens zwei Substituenten tragen. Diese Verbindungen finden im Pflanzenschutz Verwendung.

Die Veröffentlichung Kumamoto, Pharm. Bull [1966], Seiten 7 - 13 beschreibt einige substituierte Benzoylguanidine, die als Antikrebsmittel eingesetzt werden können.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoff-Mangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ -Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des Zentral-Nervensystems, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Außerdem zeichnen sich die Verbindungen durch eine Inhibition der Salzsäureproduktion der Parietalzellen des Magens aus und können somit als Arzneimittel zur Behandlung von gastrointestinalen Krankheiten verwendet werden. Derartige gastrointestinale Erkrankungen und Krankheiten der Speiseröhre sind beispielsweise Magen- und Darmulcera und die Reflux-Oesophagitis.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.
Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

### Allgemeine Vorschrift zur Herstellung von Benzoyl-guanidinen (I) aus Benzoesäuren (II, L=OH)

0,01 M des Benzoesäure-Derivates der Formel II löst bzw. suspendiert man in 60 ml wasserfreiem Tetrahydrofuran (THF) und versetzt sodann mit 1,78 g (0,011 M) Carbonyldiimidazol. Nach dem Rühren über 2 Stunden bei Zimmertemperatur werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6-7 und filtriert das entsprechende Benzoylguanidin (Formel I) ab. Die so erhaltenen Benzoylguanidine können durch Behandeln mit wäßriger oder methanolischer Salzsäure oder anderen pharmakologisch verträglichen Säuren in die entsprechenden Salze übergeführt werden.

### Beispiel 1:

3,5-Dichlorbenzoyl-guanidin-hydrochlorid
nach allgemeiner Vorschrift aus 3,5-Dichlorbenzoesäure; farblose Kristalle. Schmp. 286° C.

### Beispiel 2:

3-Chlorbenzoyl-guanidin-hydrochlorid
nach allgemeiner Vorschrift aus 3-Chlorbenzoesäure; farblose Kristalle. Schmp. 175° C.

### Beispiel 3:

3,4-Dimethylbenzoyl-guanidin-hydrochlorid
nach allgemeiner Vorschrift aus 3,4-Dimethylbenzoesäure; farblose Kristalle. Schmp. 276° C.

### Beispiel 4:

3-Trifluormethylbenzoyl-guanidin-hydrochlorid
nach allgemeiner Vorschrift aus 3-Trifluormethylbenzoesäure; farblose Kristalle. Schmp. 170° C.

### Beispiel 5:

3,5-Dichlor-4-hydroxybenzoyl-guanidin-hydrochlorid
nach allgemeiner Vorschrift aus 3,5-Dichlor-4-hydroxybenzoesäure; farblose Kristalle. Schmp. 254-256° C.

### Beispiel 6:

3,5-Di-tert.butyl-4-hydroxybenzoyl-guanidin-hydrochlorid
nach allgemeiner Vorschrift aus 3,5-Di-tert-butyl-4-hydroxy-benzoesäure; farblose Kristalle. Schmp. 163-165° C.

### Beispiel 7:

3,5-Difluorbenzoyl-guanidin-hydrochlorid
nach allgemeiner Vorschrift aus 3,5-Difluorbenzoesäure; farblose Kristalle. Schmp. 224° C.

### Beispiel 8:

4-Trifluormethylbenzoyl-guanidin-hydrochlorid
nach allgemeiner Vorschrift aus 4-Trifluormethylbenzoesäure; farblose Kristalle. Schmp. 215° C.

### Beispiel 9:

3-Chlor-5-trifluormethylbenzoyl-guanidin-hydrochlorid
nach allgemeiner Vorschrift aus 3-Chlor-5-trifluormethylbenzoesäure; farblose Kristalle. Schmp. 162° C.

### Beispiel 10:

3,5-Bis-trifluormethylbenzoyl-guanidin-hydrochlorid
nach allgemeiner Vorschrift aus 3,5-Bis-trifluormethylbenzoesäure; farblose Kristalle. Schmp. 214° C.

### Beispiel 11:

5-Trifluormethyl-3-iodbenzoyl-guanidin-hydrochlorid
nach allgemeiner Vorschrift aus 5-Trifluormethyl-3-iodbenzoesäure; farblose Kristalle. Schmp. 263° C.

### Beispiel 12:

3,5-Dimethylbenzoyl-guanidin-hydrochlorid
nach allgemeiner Vorschrift aus 3,5-Dimethylbenzoesäure; farblose Kristalle. Schmp. 216-219° C.

### Beispiel 13:

4-tert-Butylbenzoyl-guanidin-hydrochlorid
nach allgemeiner Vorschrift aus 4-tert-Butylbenzoesäure; farblose Kristalle. Schmp. 237-240° C

### Beispiel 14:

4-Chlor-3-methylbenzoyl-guanidin-hydrochlorid
nach allgemeiner Vorschrift aus 4-Chlor-3-methylbenzoesäure; farblose Kristalle. Schmp. 249-251° C.

### Beispiel 15:

3,5-Dichlor-4(4-chlorbenzyloxy)benzoyl-guanidin-hydrochlorid
nach allgemeiner Vorschrift aus 3,5-Dichlor-4(4-chlorbenzyloxy)-benzoesäure; farblose Kristalle. Schmp. 230-231° C.

3,5-Dichlor-4-(4-chlorbenzyloxy)-benzoesäure wurde durch Umsetzung von 3,5-Dichlor-4-hydroxybenzoesäure mit 4-Chlorbenzylchlorid in DMF in Gegenwart von Kaliumcarbonat bei 40° C und anschließender Hydrolyse des 3,5-Dichlor-4-(4-chlorbenzyloxy)-benzoesäure-(4-chlorbenzylesters in wäßrig-methanolischer Lösung mit NaOH und nachfolgendem Ansäuern mit 2N HCl erhalten, Schmp. 215-220° C.

### Beispiel 16:

4-tert.Butylbenzoyl-guanidin
wird erhalten durch Behandlung des entsprechenden Hydrochlorids von Beispiel 19 mit Triethylamin in Dimethylformamid und Wasser. Farblose, kristalline Substanz, Schmp. 255-258° C.

### Beispiel 17:

3,5-Dibrombenzoyl-guanidin-hydrochlorid
erhält man aus 3,5-Dibrombenzoyl-guanidin (Beispiel 28) durch Behandlung mit methanolischer Salzsäure. Farblose Kristalle. Schmp. 275° C

### Beispiel 18:

3-Azido-5-trifluormethylbenzoyl-guanidin-hydrochlorid
erhält man nach der allgemeinen Vorschrift aus 3-Azido-5-trifluormethylbenzoesäure (Fp. 123 - 125 °C), die nach Diazotierung durch Sandmeier-Reaktion aus 3-Amino-5-trifluormethylbenzoesäure und Natriumazid hergestellt wird.
Farblose kristalline Verbindung.
Fp. 197°C.

### Beispiel 19:

4-Brom-3-methylbenzoyl-guanidin-hydrochlorid
erhält man nach der allgemeinen Vorschrift aus 4-Brom-3-methylbenzoesäure.
Farblose Kristalle.
Fp. 250°C.

### Beispiel 20:

3-Chlor-4-fluor-benzoyl-guanidin-hydrochlorid
erhält man nach der allgemeinen Vorschrift aus 3-Chlor-4-fluor-benzoesäure.
Farblose Kristalle.
Fp. 188 - 189°C.

### Beispiel 21:

3,5-Di-tert.butylbenzoyl-guanidin-hydrochlorid
erhält man nach der allgemeinen Vorschrift aus 3,5-Di-tert.butylbenzoesäure.
Farblose Kristalle.
Fp. 180°C.

### Beispiel 22:

3-Brom-5-chlorbenzoyl-guanidin-hydrochlorid
erhält man nach der allgemeinen Vorschrift aus 3-Brom-5-chlorbenzoesäure.
Farblose Kristalle.
Fp. 268°C.

### Beispiel 23:

4-Brom-3-trifluormethyl-benzoylguanidin-hydrochlorid
Farblose Kristalle, Smp. 211^{o}C

Syntheseweg:
a) 4-Brom-3-trifluormethyl-benzonitril aus 4-Brom-3-trifluormethyl-anilin unter Sandmeyer-Bedingungen: Diazotierung des Amins mit Natriumnitrit in halbkonzentrierter Schwefelsäure bei 0^{o}C und anschließende Reaktion mit Cu(I)CN (aus Kupfersulfat, Natriumsulfit und NaCN) bei anfangs 0^{o}C, dann langsames Erwärmen auf Zimmertemperatur. Nach wäßrigem Aufarbeiten erfolgt Säulenchromatograpie mit Essigester/n-Heptan 2 : 8, farblose Kristalle, Smp. 77-80^{o}C
b) 4-Brom-3-trifluormethyl-benzoesäure aus a) durch säurekatalysierte Hydratisierung mittels Eisessig/konzentrierter Salzsäure am Rückfluß für 4 Stunden, wäßrige Aufarbeitung, farblose Kristalle, Smp. 177 - 80^{o}C
c) 4-Brom-3-trifluormethyl-benzoylguanidin-hydrochlorid aus b) nach allgemeiner Vorschrift

### Beispiel 24:

4-Isopropyl-3-trifluormethyl-benzoylguanidin-hydrochlorid
Farblose Kristalle, Smp. 213 - 14°C

Syntheseweg:
a) 4-Brom-3-trifluormethyl-benzoesäuremethylester aus 4-Brom-3-trifluormethylbenzoesäure (23b) durch Erhitzen in Methanol in Gegenwart von Acetylchlorid, wäßrige Aufarbeitung, farblose Kristalle, Smp. 56 - 57°C
b) 4-lsopropyl-3-trifluormethyl-benzoesäuremethylester aus 4-Brom-3-trifluormethyl-benzoesäuremethylester (a) durch cross-coupling mit 1.5 Äquivalenten Isopropylzinkchlorid (aus Isopropylmagnesiumchlorid durch Transmetallierung mit Zink(II)chlorid-Etherat in THF) durch Rühren bei Zimmertemperatur in Gegenwart von katalytischen Mengen Palladium(II)[1,1'-bis-(diphenylphosphino)-ferrocen]chlorid und Kupfer(I)iodid, wäßrige Aufarbeitung, Extraktion mit Essigester und anschließende Säulenchromatographie an Kieselgel mit Essigester/Cyclohexan (2 : 8), farbloses Öl.
c) 4-Isopropyl-3-trifluormethyl-benzoylguanidin-hydrochlorid durch Erhitzen zum Sieden in THF in Gegenwart von Guanidin und anschließende Hydrochloridbildung

### Beispiel 25 :

4-Cyclopentyl-3-trifluormethyl-benzoylguanidin-hydrochlorid
Farblose Kristalle, Smp. 229 - 31°C

Syntheseweg:
a) 4-Cyclopentyl-3-trifluormethyl-benzoesäuremethylester aus 4-Brom-3-trifluormethyl-benzoesäuremethylester (24a) durch cross-coupling mit Cyclopentylzinkchlorid analog Beispiel 24b), farbloses Öl
b) 4-Cyclopentyl-3-trifluormethyl-benzoylguanidin-hydrochlorid analog 24c).

### Beispiel 26:

3-Methyl-5-trifluormethyl-benzoylguanidin-hydrochlorid
Farblose Kristalle, Smp.181 - 82°C

### Syntheseweg:

a) 3-lod-5-trifluormethyl-benzoesäure-methylester aus 3-lod-5-trifluormethylbenzoesäure analog Beispiel 24a), farbloses Öl
b) 3-Methyl-5-trifluormethyl-benzoesäuremethylester aus 3-lod-5-trifluormethylbenzoesäure-methylester durch cross-coupling mit Methylzinkchlorid analog Beispiel 24b), farbloses Öl
b) 3-Methyl-5-trifluormethyl-benzoylguanidin-hydrochlorid analog 24c)

### Beispiel 27:

3-lsopropyl-5-trifluormethyl-benzoylguanidin-hydrochlorid
Farblose Kristalle, Smp. 110 - 12°C

### Syntheseweg:

a) 3-lsopropyl-5-trifluormethyl-benzoesäuremethylester aus 3-lod-5-trifluormethyl-benzoesäuremethylester (26a) durch cross-coupling mit Isopropylzinkchlorid analog 24b), farbloses Öl
b) 3-lsopropyl-5-trifluormethyl-benzoylguanidin-hydrochlorid analog 24c)

### Beispiel 28:

3-Cyclopentyl-5-trifluormethyl-benzoylguanidin-hydrochlorid
Farblose Kristalle, Smp. 110^{o}C unter Zersetzung

### Syntheseweg:

a) 3-Cyclopentyl-5-trifluormethyl-benzoesäuremethylester aus 3-Iod-5-trifluormethyl-benzoesäuremethylester (26a) (durch cross-coupling mit Cyclopentylzinkchlorid analog 24b), farbloses Öl
b) 3-Cyclopentyl-5-trifluormethyl-benzoylguanidin-hydrochlorid analog 24c)

### Beispiel 29 :

3-Phenyl-5-trifluormethyl-benzoylguanidin-hydrochlorid
Farblose Kristalle, Smp. 217 - 21^{o}C

### Syntheseweg:

a) 3-Phenyl-5-trifluormethyl-benzoesäuremethylester aus 3-Iod-5-trifluormethyl-benzoesäuremethylester (26a) durch cross-coupling mit 1.1 Äquivalenten Phenylboronsäure in wäßrigem Methanol/Toluol-Gemisch (Rückfluß, 4 Stunden) in Gegenwart von katalytischen Mengen Palladium-acetat, Triphenylphosphin und Natriumcarbonat, Lösungsmittel abdestillieren, in Essigester aufnehmen, mit verdünnter Salzsäure neutral stellen, nach wäßriger Aufarbeitung Säulenchromatographie an Kieselgel mit Essigester/Cyclohexan (3 : 7), farbloses Öl
b) 3-Phenyl-5-trifluormethyl-benzoylguanidin-hydrochlorid analog 24c)

### Beispiel 30:

4-Fluor-3-trifluormethyl-benzoylguanidin-hydrochlorid
Farblose Kristalle, Smp. 159 - 60
aus 4-Fluor-3-trifluormethyl-benzoesäure nach allgemeiner Vorschrift

### Beispiel 31:

4-Phenoxy-3-trifluormethyl-benzoylguanidin-hydrochlorid
Farblose Kristalle, Smp. 162 - 65^{o}C
aus 4-Fluor-3-trifluormethyl-benzoylguanidin (30-Base) durch Reaktion mit Phenol in Gegenwart von Kaliumcarbonat in DMF bei 120^{o}C, wäßrige Aufarbeitung, Säulenchromatographie, Methylenchlorid/Methanol 9 : 1, anschließende Hydrochloridbildung

### Beispiel 32:

4-(4-Fluor-phenoxy)-3-trifluormethyl-benzoylguanidin-hydrochlorid
Farblose Kristalle, Smp. 165 - 67^{o}C
aus (30-Base) analog Verfahren (31) mittels 4-Fluor-phenol

### Beispiel 33:

4-(4-Chlor-phenoxy)-3-trifluormethyl-benzoylguanidin-hydrochlorid
Farblose Kristalle, Smp. 195 - 97^{o}C
aus (30-Base) analog Verfahren (31) mittels 4-Chlor-phenol

### Beispiel 34:

5-Fluor-3-trifluormethyl-benzoylguanidin-hydrochlorid
Farblose Kristalle, Smp. 150 - 51^{o}C
aus 5-Fluor-3-trifluormethyl-benzoesäure nach allgemeiner Vorschrift

### Beispiel 35:

4-Phenylethinyl-3-trifluormethyl-benzoylguanidin-hydrochlorid
Farblose Kristalle, Smp. 150^{o}C unter Zersetzung

Syntheseweg:
a) 4-Phenylethinyl-3-trifluormethyl-benzoesäuremethylester aus 4-Brom-3-trifluormethyl-benzoesäure-methylester (24a) durch Stephans-Castro-coupling mit 2.5 Äquivalenten Phenylacetylen, Rühren bei Zimmertemperatur für 24 Stunden in Gegenwart von katalytischen Mengen (5 mol%) Bis-(Triphenylphosphin)-palladium(II)chlorid, 15 mol% Kupfer(I)iodid und 3 Äquivalenten n-Butylamin, wäßrige Ammoniumchloridaufarbeitung, Extraktion mit Essigester und anschließende Säulenchromatographie an Kieselgel mit Essigester/Cyclohexan (3 : 7), leicht bräunliches Öl.
b) 4-Phenylethinyl-3-trifluormethyl-benzoylguanidin-hydrochlorid analog 24c)

### Beispiel 36:

3-Brom-4-methylbenzoyl-guanidin-hydrochlorid
erhält man analog der allgemeinen Vorschrift aus 3-Brom-4-methylbenzoesäure.
Farblose Kristalle.
Fp. 250°C.

### Beispiel 37:

3-Chlor-4-isopropylbenzoyl-guanidin-hydrochlorid
erhält man analog der allgemeinen Vorschrift aus 3-Chlor-4-isopropylbenzoesäure.
Farblose Kristalle.
Fp. 185 °C.

### Beispiel 38:

3,4,5-Trichlorbenzoyl-guanidin-hydrochlorid
erhält man analog der allgemeinen Vorschrift aus 3,4,5-Trichlorbenzoesäure.
Farblose Kristalle.
Fp. 194 °C.

### Beispiel 39:

3-Brom-5-methylbenzoyl-guanidin-hydrochlorid
erhält man analog der allgemeinen Vorschrift aus 3-Brom-5-methylbenzoesäure.
Farblose Kristalle.
Fp. 235 - 236 °C.

### Beispiel 40:

4-Chlor-3,5-dimethylbenzoyl-guanidin-hydrochlorid
erhält man analog der allgemeinen Vorschrift aus 4-Chlor-3,5-dimethylbenzoesäure.
Farblose Kristalle.
Fp. 244 - 247 °C.

### Beispiel 41:

3-Trifluormethyloxybenzoyl-guanidin-hydrochlorid
erhält man analog der allgemeinen Vorschrift aus 3-Trifluormethyloxybenzoesäure.
Kristalle.
Fp. 146 - 148 °C.

### Beispiel 42:

4-Trifluormethyloxybenzoyl-guanidin-hydrochlorid
erhält man analog der allgemeinen Vorschrift aus 4-Trifluormethyloxybenzoesäure.
Kristalle.
Fp. 259 °C.

### Beispiel 43:

4-Cyclohexylbenzoyl-guanidin-hydrochlorid
erhält man analog der allgemeinen Vorschrift aus 4-Cyclohexylbenzoesäure.
Kristalle.
Fp. 273 °C.

### Beispiel 44:

3-Chlor-4-cyclopentyloxybenzoyl-guanidin-hydrochlorid
erhält man analog der allgemeinen Vorschrift aus 3-Chlor-4-cyclopentyloxybenzoesäure. Farblose Kristalle.
Fp. 273 °C.

Die 3-Chlor-4-cyclopentyloxybenzoesäure vom Schmp. 148 - 151 °C erhält man durch Hydrolyse des 3-Chlor-4-cyclopentyloxy-benzoesäuremethylesters (amorphe ölartige Substanz) in einem Gemisch aus wäßriger NaOH und Dioxan und anschließendem Azidifizieren der alkalischen Hydrolyselösung mit halbkonzentrierter Salzsäure.
Der 3-Chlor-4-cyclopentyloxy-benzoesäuremethylester wird erhalten durch Kochen von 3-Chlor-4-hydroxybenzoesäuremethylester und Jodcyclopentan in Aceton in Gegenwart von überschüßigem festen gemahlenen Kaliumcarbonat. Nach Verdampfen des Acetons wird der ölige Rückstand mit Wasser aufgenommen, anschließend mit Ethylacetat extrahiert und das Lösungsmittel nach Trocknen über Natriumsulfat verdampft.

### Beispiel 45:

3-Isopropyl-4-methoxybenzoyl-guanidin-hydrochlorid
erhält man analog der allgemeinen Vorschrift aus 3-Isopropyl-4-methoxybenzoesäure. Farblose Kristalle.
Fp. 214 °C.

### Beispiel 46:

3-Chlor-4-cyclooctyloxybenzoyl-guanidin-hydrochlorid
erhält man analog der allgemeinen Vorschrift aus 3-Chlor-4-cyclooctyloxybenzoesäure. Farblose Kristalle.
Fp. 243 °C.

Die 3-Chlor-4-cyclooctyloxybenzoesäure vom Schmp. 110-112 °C erhält man durch Hydrolyse des 3-Chlor-4-cyclooctyloxy-benzoesäuremethylesters (amorphe ölartige Substanz) in einem Gemisch aus wäßriger NaOH und Methanol und anschließendem Azidifizieren der alkalischen Hydrolyselösung mit halbkonzentrierter Salzsäure.
Der 3-Chlor-4-cyclooctyloxy-benzoesäuremethylester wird erhalten durch Erhitzen von 3-Chlor-4-hydroxybenzoesäuremethylester und Cyclooctylbromid in Dimethylformamid über 20 Stunden in Gegenwart von überschüßigem festen gemahlenen Kaliumcarbonat. Nach Verdampfen des Lösungsmittels wird der ölige Rückstand mit Wasser versetzt , anschließend mit Ethylacetat extrahiert und das Lösungsmittel nach Trocknen über Natriumsulfat verdampft.

### Beispiel 47:

4-tert.Butyl-3-methoxybenzoyl-guanidin-hydrochlorid
erhält man analog der allgemeinen Vorschrift aus 4-tert.Butyl-3-methoxybenzoesäure. Farblose Kristalle.
Fp. 227-231 °C.

Die verwendete 4-tert.Butyl-3-methoxybenzoesäure erhält man durch Oxidation von 4-tert.Butyl-3-methoxytoluol in wäßrig alkalischer Kaliumpermanganatlösung.

### Beispiel 48:

3-Brom-4-fluorbenzoyl-guanidin-hydrochlorid
erhält man analog der allgemeinen Vorschrift aus 3-Brom-4-fluorbenzoesäure.
Farblose Kristalle.
Fp. 215 °C.

### Beispiel 49:

3-tert.Butyl-4-hydroxybenzoyl-guanidin-hydrochlorid
erhält man analog der allgemeinen Vorschrift aus 3-tert.Butyl-4-hydroxybenzoesäure. Farblose Kristalle.
Fp. 216 °C.

### Beispiel 50:

3-Cyano-4-methoxybenzoyl-guanidin-hydrochlorid
erhält man analog der allgemeinen Vorschrift aus 3-Cyano-4-methoxybenzoesäure.
Farblose Kristalle.
Fp. 236 °C.

### Beispiel 51:

3-tert. Butyl-4-methoxybenzoyl-guanidin-hydrochlorid
erhält man analog der allgemeinen Vorschrift aus 3-tert.Butyl-4-methoxybenzoesäure. Farblose Kristalle.
Fp. 260 - 62 °C.

### Beispiel 52:

3-Chlor-4-(1-hexyl)benzoyl-guanidin-hydrochlorid
erhält man analog der allgemeinen Vorschrift aus 3-Chlor-4-(1-hexyl)benzoesäure. Farblose Kristalle.
Fp. 286 °C (Zers.).

### Beispiel 53:

3-tert.Butyl-4-(2-methyl-1-propyl)benzoyl-guanidin-hydrochlorid
erhält man analog der allgemeinen Vorschrift aus 3-tert.Butyl-4-(2-methyl-1-propyl)benzoesäure. Farblose Kristalle.
Fp. 218 - 228 °C (Zers.).

### Beispiel 54:

4-lsopropyl-3-pentafluorethylbenzoyl-guanidin-hydrochlorid
erhält man analog der allgemeinen Vorschrift aus 4-lsopropyl-3-pentafluorethylbenzoesäure. Farbloser, amorpher Feststoff.

### Beispiel 55:

3-tert. Butyl-4-isopropylbenzoyl-guanidin-hydrochlorid
erhält man analog der allgemeinen Vorschrift aus 3-tert.Butyl-4-isopropylbenzoesäure. Farblose Kristalle.
Fp. 145 - 165 °C.

### Beispiel 56:

4-Isopropylbenzoyl-guanidin-hydrochlorid
erhält man analog der allgemeinen Vorschrift aus Isopropylbenzoesäure. Farblose Kristalle.
Fp. 193 - 198 °C.

### Beispiel 57:

3-Trifluormethylbenzoyl-guanidin
erhält man analog der allgemeinen Vorschrift aus 3-Trifluorbenzoesäure. Farblose amorph-ölige Masse.

### Beispiel 58:

3-Trifluormethylbenzoyl-guanidin-methansulfonat
erhält man analog der allgemeinen Vorschrift aus 3-Trifluormethylbenzoyl-guanidin durch Behandeln mit Methansulfonsäure in Ethylacetat. Farblose Kristalle.
Fp. 167 - 170 °C.

### Beispiel 59:

4-Fluor-3-isobutylbenzoyl-guanidin-hydrochlorid
erhält man analog der allgemeinen Vorschrift aus 4-Fluor-3-isobutylbenzoyl-guanidin.
Fp. 136-140°C.

## Patentansprüche

1. Benzoylguanidin der Formel I 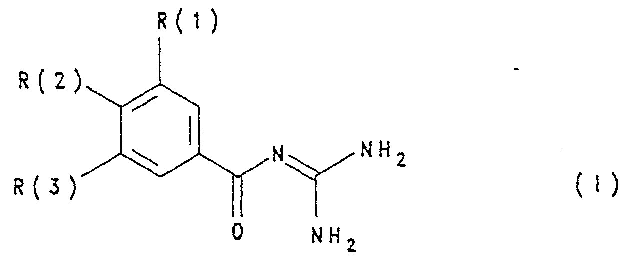 worin bedeuten:
R(1), R(2), R(3)
Wasserstoff, F, Cl, Br, I, (C₁-C₁₂)-Alkyl, einer der Substituenten R(1), R(2) oder R(3)
N₃, CN, OH oder (C₁-C₁₀)-Alkyloxy, wenn mindestens einer der restlichen Substituenten R(1), R(2) oder R(3) einen hinreichend lipophilen Alkylrest mit 3 bis 12 Kohlenstoff-Atomen bedeutet,
oder einer der Substituenten R(1), R(2) oder R(3) ist
R(4)-CₙH₂ₙ-Oₘ- mit
m gleich Null oder 1,
n gleich Null, 1, 2 oder 3,
R(4) gleich CₚF₂ₚ₊₁
mit p gleich 1, 2 oder 3, sofern n gleich Null oder ist, oder
R(4) gleich (C₃-C₁₂)-Cycloalkyl, Phenyl, wobei Phenyl unsubstituiert ist oder substituiert mit einem Substituenten aus der
Gruppe
F, Cl, CF₃, Methyl, Methoxy, NR(5)R(6),
mit R(5) und R(6) gleich Wasserstoff oder (C₁-C₄)-Alkyl,
oder einer der Substituenten R(1), R(2) oder R(3)
-C≡CR(5), -C[R(6)] = CR(5),
R(5) Phenyl,
das unsubstituiert oder substituiert ist mit 1 - 3 Substituenten aus der Gruppe F, Cl, CF₃, Methyl, Methoxy, Hydroxy, Amino, Methylamino oder Dimethylamino,
(C₁-C₉)-Heteroaryl,
das unsubstituiert oder wie Phenyl substituiert ist,
(C₁-C₆)-Alkyl,
das unsubstituiert oder mit 1 - 3 OH substituiert ist,
(C₃-C₈)-Cycloalkyl,
R(6) Wasserstoff, Methyl,
sowie deren pharmakologisch akzeptable Salze,
mit Ausnahme der Verbindungen Benzoyl-guanidin, 4-Chlorbenzoyl-guanidin, 3,4-Dichlorbenzoyl-guanidin; 3- oder 4-Methylbenzoyl-guanidin.

2. Benzoylguanidin der Formel I nach Anspruch 1, in der bedeuten:
R(1), R(2), R(3)
Wasserstoff, F, Cl, Br, (C₁-C₈)-Alkyl,
einer der Substituenten R(1), R(2) oder R(3)
OH oder (C₁-C₆)-Alkyloxy, wenn mindestens einer der restlichen Substituenten R(1), R(2) oder R(3) einen hinreichend lipophilen Alkylrest mit 3 bis 6 Kohlenstoff-Atomen bedeutet,
oder einer der Substituenten R(1), R(2) oder R(3) ist
R(4)-CₙH₂ₙ-Oₘ- mit
m gleich Null oder 1,
n gleich Null, 1, 2 oder 3,
R(4) gleich CₚF₂ₚ₊₁
mit p gleich 1, sofern n gleich Null oder 1 ist, oder
R(4) gleich (C₅-C₇)-Cycloalkyl oder Phenyl, wobei Phenyl unsubstituiert ist oder substituiert mit einem Substituenten aus der Gruppe
F, Cl, CF₃, Methyl, Methoxy,
oder einer der Substituenten R(1), R(2) oder R(3)
-C≡CR(5),
R(5) Phenyl, (C₁-C₄)-Alkyl,
das unsubstituiert oder mit einem OH substituiert ist,
sowie deren pharmakologisch akzeptable Salze,
mit Ausnahme der Verbindungen Benzoyl-guanidin, 4-Chlorbenzoyl-guanidin, 3,4-Dichlorbenzoyl-guanidin, 3- oder 4-Methylbenzoyl-guanidin.

3. Benzoylguanidin der Formel I nach Anspruch 1, ausgewählt aus der Gruppe bestehend aus 3-Trifluormethylbenzoyl-guanidin-hydrochlorid, 3,5-Bistrifluormethylbenzoyl-guanidin-hydrochlorid, 3-Methyl-5-trifluormethyl-benzoylguanidin-hydrochlorid, 4-Fluor-3-trifluormethyl-benzoylguanidin-hydrochlorid, 4-(4-Fluor-phenoxy)-3-trifluormethyl-benzoylguanidin-hydrochlorid, 5-Fluor-3-trifluormethyl-benzoylguanidin-hydrochlorid, 3-Chlor-4-isopropylbenzoyl-guanidin-hydrochlorid, 4-tert.Butyl-3-methoxybenzoyl-guanidin-hydrochlorid, 3-tert.Butyl-4-hydroxybenzoyl-guanidin-hydrochlorid, 3-tert.Butyl-4-isopropylbenzoyl-guanidin-hydrochlorid.

4. Verfahren zur Herstellung einer Verbindung I nach Anspruch 1, dadurch gekennzeichnet, daß man eine Verbindung der Formel II 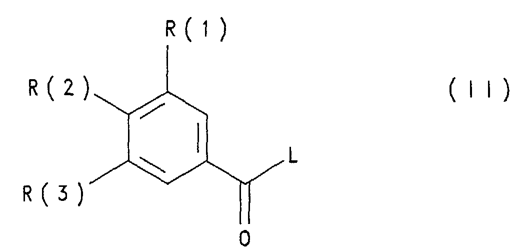 mit Guanidin umsetzt, worin R(1) bis R(3) die angegebene Bedeutung besitzen und L für eine leicht nucleophil substituierbare leaving group steht.

5. Verwendung einer Verbindung I nach Anspruch 1 zur Herstellung eines Medikaments zur Behandlung von Arrhythmien.

6. Verwendung einer Verbindung I nach Anspruch 1 zur Herstellung eines Medikaments zur Behandlung von akuten oder chronischen, durch Ischämien ausgelösten Schäden oder durch Ischämie primär oder sekundär induzierter Krankheiten.

7. Verwendung einer Verbindung I nach Anspruch 1 zur Herstellung eines Medikaments zur Behandlung von Zuständen nach Organtransplantationen, zur Protektion der Organe des Spenders und des Empfängers vor und während der Entnahme und Übertragung auf den Empfänger.

8. Verwendung einer Verbindung I nach Anspruch 1 zur Herstellung eines Medikaments zur Protektion von Organen bei deren Lagerung außerhalb von lebenden Organismen.

9. Verwendung einer Verbindung I nach Anspruch 1 zur Herstellung eines Medikaments zur Protektion bei der Durchführung angioplastischer Eingriffe.

10. Verwendung einer Verbindung I nach Anspruch 1 zur Herstellung eines Medikaments zur Behandlung des Schlaganfalls und des Hirnödems.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zur Behandlung der Atherogenese und Atherosklerose.

13. Verwendung einer Verbindung I nach Anspruch 1 zur Herstellung eines Medikaments zur Behandlung diabetischer Spätkomplikationen.

14. Verwendung einer Verbindung I nach Anspruch 1 zur Herstellung eines Medikaments zur Behandlung von Krebs.

15. Verwendung einer Verbindung I nach Anspruch 1 zur Herstellung eines Medikaments zur Behandlung von fibrotischen Krankheiten.

16. Verwendung einer Verbindung I nach Anspruch 1 zur Herstellung eines Medikaments zur Behandlung von Organhypertrophien und -hyperplasien.

17. Verwendung einer Verbindung I nach Anspruch 1 als wissenschaftliche tools und Diagnostika.

18. Verwendung einer Verbindung I nach Anspruch 1 zur Herstellung eines Medikaments zur Verhinderung der Genese des Bluthochdrucks.

19. Verwendung einer Verbindung I nach Anspruch 1 zur Herstellung eines Medikaments zur Behandlung von Erkrankungen, die durch proliferative Prozesse ausgelöst werden.

20. Verwendung einer Verbindung I nach Anspruch 1 zur Herstellung eines Medikaments zur Behandlung gastrointestinaler Erkrankungen und Erkrankungen der Speiseröhre, die durch eine Hypersekretion von Magensäure hervorgerufen werden.

21. Heilmittel, gekennzeichnet durch einen wirksamen Gehalt an einer Verbindung I nach Anspruch 1.

## Claims

1. A benzoylguanidine of the formula I 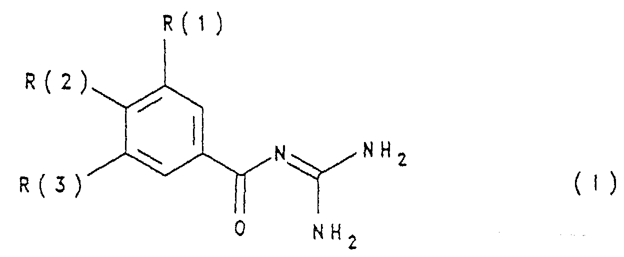 in which:
R(1), R(2), R(3) are hydrogen, F, Cl, Br, I or (C₁-C₁₂)-alkyl,
one of the substituents R(1), R(2) or R(3) is N₃, CN, OH or (C₁-C₁₀)-alkyloxy, if at least one of the remaining substituents R(1), R(2) or R(3) is a sufficiently lipophilic alkyl radical having 3 to 12 carbon atoms,
or one of the substituents R(1), R(2) or R(3) is
R(4) -CₙH₂ₙ-Oₘ- where
m is zero or 1,
n is zero, 1, 2 or 3,
R(4) is CₚF₂ₚ₊₁
where p is 1, 2 or 3 if n is zero or 1, or
R(4) is (C₃-C₁₂)-cycloalkyl or phenyl, phenyl being unsubstituted or substituted by a substituent selected from the group consisting of
F, Cl, CF₃, methyl, methoxy or NR(5)R(6),
where R(5) and R(6) are hydrogen or (C₁-C₄)-alkyl,
or one of the substituents R(1), R(2) or R(3) is -C≡CR(5), -C[R(6)] = CR(5),
R(5) is phenyl
which is unsubstituted or substituted by 1-3 substituents selected from the group consisting of F, Cl, CF₃, methyl, methoxy, hydroxyl, amino, methylamino or dimethylamino,
(C₁-C₉)-heteroaryl,
which is unsubstituted or substituted as phenyl,
(C₁-C₆)-alkyl,
which is unsubstituted or substituted by 1-3 OH, or
(C₃-C₈)-cycloalkyl,
R(6) is hydrogen or methyl,
and the pharmacologically acceptable salts thereof,
with the exception of the compounds benzoylguanidine, 4-chlorobenzoylguanidine, 3,4-dichlorobenzoylguanidine and 3- or 4-methylbenzoylguanidine.

2. A benzoylguanidine of the formula I as claimed in claim 1, in which:
R(1), R(2), R(3) are
hydrogen, F, Cl, Br or (C₁-C₈)-alkyl,
one of the substituents R(1), R(2) or R(3) is
OH or (C₁-C₆)-alkyloxy, if at least one of the remaining substituents R(1), R(2) or R(3) is a sufficiently lipophilic alkyl radical having 3 to 6 carbon atoms,
or one of the substituents R(1), R(2) or R(3) is
R(4)-CₙH₂ₙ-Oₘ- where
m is zero or 1,
n is zero, 1, 2 or 3,
R(4) is CₚF₂ₚ₊₁
where p is 1 if n is zero or 1, or
R(4) is (C₅-C₇)-cycloalkyl or phenyl, phenyl being unsubstituted or substituted by a substituent selected from the group consisting of
F, Cl, CF₃, methyl or methoxy,
or one of the substituents R(1), R(2) or R(3) is
-C≡CR(5),
R(5) is phenyl or (C₁-C₄)-alkyl,
which is unsubstituted or substituted by an OH,
and the pharmacologically acceptable salts thereof,
with the exception of the compounds benzoylguanidine, 4-chlorobenzoylguanidine, 3,4-dichlorobenzoylguanidine and 3- or 4-methylbenzoylguanidine.

3. A benzoylguanidine of the formula I as claimed in claim 1, selected from the group comprising 3-trifluoromethylbenzoylguanidine hydrochloride, 3,5-bis-trifluoromethylbenzoylguanidine hydrochloride, 3-methyl-5-trifluoromethylbenzoylguanidine hydrochloride, 4-fluoro-3-trifluoromethylbenzoylguanidine hydrochloride, 4-(4-fluorophenoxy)-3-trifluoromethylbenzoylguanidine hydrochloride, 5-fluoro-3-trifluoromethylbenzoylguanidine hydrochloride, 3-chloro-4-isopropylbenzoylguanidine hydrochloride, 4-tert-butyl-3-methoxybenzoylguanidine hydrochloride, 3-tert-butyl-4-hydroxybenzoylguanidine hydrochloride and 3-tert-butyl-4-isopropylbenzoylguanidine hydrochloride.

4. A process for the preparation of a compound I as claimed in claim 1, which comprises reacting a compound of the formula II 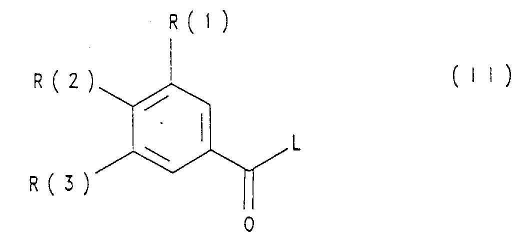 in which R(1) to R(3) have the abovementioned meaning and L is a leaving group which can readily be substituted by a nucleophile, with guanidine.

5. The use of a compound I as claimed in claim 1 for the preparation of a pharmaceutical for the treatment of arrhythmias.

6. The use of a compound I as claimed in claim 1 for the preparation of a pharmaceutical for the treatment of acute or chronic, ischemia-triggered types of damage or diseases which are primarily or secondarily induced by ischemia.

7. The use of a compound I as claimed in claim 1 for the preparation of a pharmaceutical for the treatment of conditions after organ transplants, and for protecting the organs of the donor and the acceptor before and during removal and transfer to the acceptor.

8. The use of a compound I as claimed in claim 1 for the preparation of a pharmaceutical for protecting organs when they are stored outside live organisms.

9. The use of a compound I as claimed in claim 1 for the preparation of a pharmaceutical for protective purposes when carrying out invasive angioplastic treatment.

10. The use of a compound I as claimed in claim 1 for the preparation of a pharmaceutical for the treatment of apoplexy and of cerebral edema.

11. The use of a compound I as claimed in claim 1 for the preparation of a pharmaceutical for the treatment of shock conditions.

12. The use of a compound I as claimed in claim 1 for the preparation of a pharmaceutical for the treatment of atherogenesis and atherosclerosis.

13. The use of a compound I as claimed in claim 1 for the preparation of a pharmaceutical for the treatment of late complications in diabetes.

14. The use of a compound I as claimed in claim 1 for the preparation of a pharmaceutical for the treatment of cancer.

15. The use of a compound I as claimed in claim 1 for the preparation of a pharmaceutical for the treatment of fibrotic diseases.

16. The use of a compound I as claimed in claim 1 for the preparation of a pharmaceutical for the treatment of organ hypertrophias and hyperplasias.

17. The use of a compound I as claimed in claim 1 as scientific tools and diagnostics.

18. The use of a compound I as claimed in claim 1 for the preparation of a pharmaceutical for preventing the genesis of hypertension.

19. The use of a compound I as claimed in claim 1 for the preparation of a pharmaceutical for the treatment of disorders triggered by proliferative processes.

20. The use of a compound I as claimed in claim 1 for the preparation of a pharmaceutical for the treatment of gastrointestinal disorders and disorders of the esophagus caused by hypersecretion of gastric acid.

21. Medicament which comprises an effective amount of a compound I as claimed in claim 1.

## Revendications

1. Benzoylguanidine de formule I 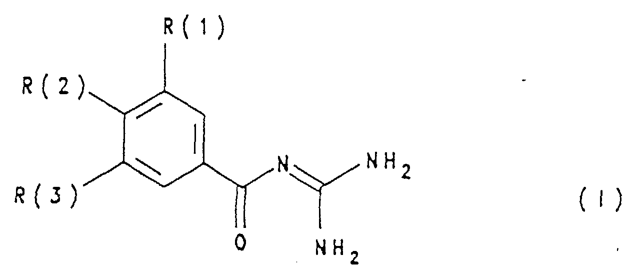 dans laquelle :
R(1), R(2), R(3) représentent
un atome d'hydrogène, F, Cl, Br, I, un radical alkyle en C₁-C₁₂,
un des substituants R(1), R(2) ou R(3) représente
N₃, CN, OH, ou un radical alkyloxy en C₁-C₁₀, si au moins un des substituants restants R(1), R(2) ou R(3) représente un résidu alkyle suffisamment lipophile avec de 3 jusqu'à 12 atomes de carbone,
ou un des substituants R(1), R(2) ou R(3) est
R(4) -CₙH₂ₙ-Oₘ- avec
m égal à zéro ou 1,
n égal à zéro, 1, 2 ou 3,
R(4) est CₚF₂ₚ₊₁
avec p égal à 1, 2 ou 3, si n est égal à zéro ou 1, ou
R(4) est un radical cylcloalkyle en C₃-C₁₂, phényle, le radical phényle étant non substitué ou substitué avec un substituant du groupe
F, Cl, CF₃, méthyle, méthoxy, NR(5)R(6), avec R(5) et R(6) égaux à un atome d'hydrogène ou un radical alkyle en C₁-C₄,
ou un des substituants R(1), R(2) ou R(3) représente -C≡CR(5), -C[R(6)]=CR(5),
R(5) représentant un radical phényle,
qui est non substitué ou substitué avec 1 - 3 substituants du groupe F, Cl, CF₃, méthyle, méthoxy, hydroxy, amino, méthylamino ou diméthylamino,
un radical hétéroaryle en C₁-C₉,
qui est non substitué ou substitué comme le groupe phényle,
un radical alkyle en C₁-C₆,
qui est non substitué ou substitué avec 1 - 3 groupes OH,
un radical cycloalkyle en C₃-C₈,
R(6) représentant un atome d'hydrogène, un groupe méthyle,
ainsi que leurs sels pharmacologiquement acceptables,
à l'exclusion des composés benzoyl-guanidine, 4-chlorobenzoyl-guanidine, 3,4-dichlorobenzoyl-guanidine, 3- ou 4-méthylbenzoyl-guanidine.

2. Benzoylguanidine de formule I selon la revendication 1, dans laquelle :
R(1), R(2), R(3) représentent
un atome d'hydrogène, F, Cl, Br, un radical alkyle en C₁-C₈,
un des substituants R(1), R(2) ou R(3) représente
un groupe OH, ou un radical alkyloxy en C₁-C₆, si au moins un des substituants restants R(1), R(2) ou R(3) représente un résidu alkyle suffisamment lipophile avec de 3 jusqu'à 6 atomes de carbone,
ou un des substituants R(1), R(2) ou R(3) est
R(4) -CₙH₂ₙ-Oₘ- avec
m égal à zéro ou 1,
n égal à zéro, 1, 2 ou 3,
R(4) est CₚF₂ₚ₊₁
avec p égal à 1, si n est égal à zéro ou 1, ou
R(4) est un radical cylcloalkyle en C₅-C₇ ou phényle, le radical phényle étant non substitué ou substitué avec un substituant du groupe
F, Cl, CF₃, méthyle, méthoxy,
ou un des substituants R(1), R(2) ou R(3) représente -C≡CR(5),
R(5) représentant un radical phényle, alkyle en C₁-C₄,
qui est non substitué ou substitué avec 1 groupe OH,
ainsi que leurs sels pharmacologiquement acceptables,
à l'exclusion des composés benzoyl-guanidine, 4-chlorobenzoyl-guanidine, 3,4-dichlorobenzoyl-guanidine, 3- ou 4-méthylbenzoyl-guanidine.

3. Benzoylguanidine de formule I selon la revendication 1, choisie dans le groupe constitué du chlorhydrate de 3-trifluorométhylbenzoyl-guanidine, du chlorhydrate de 3,5-bis-trifluorométhylbenzoyl-guanidine, du chlorhydrate de 3-méthyl-5-trifluorométhylbenzoyl-guanidine, du chlorhydrate de 4-fluoro-3-trifluorométhyl-benzoylguanidine, du chlorhydrate de 4(4-fluoro-phénoxy)-3-trifluorométhyl-benzoylguanidine, du chlorhydrate de 5-fluoro -3-trifluorométhylbenzoylguanidine, du chlorhydrate de 3-chloro-4-isopropyl-benzoylguanidine, du chlorhydrate de 4-tert.-butyl-3-méthoxybenzoyl-guanidine, du chlorhydrate de 3-tert.-butyl-4-hydroxybenzoyl-guanidine, du chlorhydrate de 3-tert.-butyl-4-isopropylbenzoyl-guanidine.

4. Procédé de préparation d'un composé I selon la revendication, caractérisé en ce que l'on fait réagir un composé de formule II 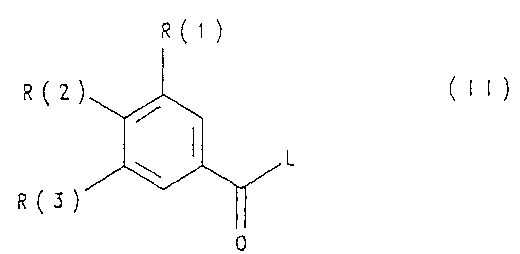 avec de la guanidine, formule II dans laquelle R(1) à R(3) possèdent la signification indiquée et L représente un groupe partant substituable par substitution nucléophile.

5. Utilisation d'un composé I selon la revendication 1 pour la préparation d'un médicament pour le traitement des arythmies.

6. Utilisation d'un composé I selon la revendication 1 pour la préparation d'un médicament pour le traitement des dommages aigus ou chroniques dus aux ischémies ou les maladies induites de manière primaire ou secondaire par l'ischémie.

7. Utilisation d'un composé I selon la revendication 1 pour la préparation d'un médicament pour le traitement des états après transplantation d'organes, pour la protection des organes du donneur et du receveur avant et pendant le prélèvement et la transplantation chez le receveur.

8. Utilisation d'un composé I selon la revendication 1 pour la préparation d'un médicament pour la protection d'organes lors de leur stockage en dehors des organismes vivants.

9. Utilisation d'un composé I selon la revendication 1 pour la préparation d'un médicament pour la protection lors de la réalisation d'angioplasties.

10. Utilisation d'un composé I selon la revendication 1 pour la préparation d'un médicament pour le traitement de l'attaque d'apoplexie et de l'oedème du cerveau.

11. Utilisation d'un composé I selon la revendication 1 pour la préparation d'un médicament pour le traitement des états de choc.

12. Utilisation d'un composé I selon la revendication 1 pour la préparation d'un médicament pour le traitement de l'athérogenèse et de l'athérosclérose.

13. Utilisation d'un composé I selon la revendication 1 pour la préparation d'un médicament pour le traitement des complications tardives du diabète.

14. Utilisation d'un composé I selon la revendication 1 pour la préparation d'un médicament pour le traitement du cancer.

15. Utilisation d'un composé I selon la revendication 1 pour la préparation d'un médicament pour le traitement des maladies fibreuses.

16. Utilisation d'un composé I selon la revendication 1 pour la préparation d'un médicament pour le traitement des hypertrophies et des hyperplasies d'organes.

17. Utilisation d'un composé I selon la revendication 1 comme outil scientifique et comme moyen de diagnostic.

18. Utilisation d'un composé I selon la revendication 1 pour la préparation d'un médicament pour empêcher la genèse de l'hypertension.

19. Utilisation d'un composé I selon la revendication 1 pour la préparation d'un médicament pour le traitement de maladies qui sont induites par des processus de type prolifératif.

20. Utilisation d'un composé I selon la revendication 1 pour la préparation d'un médicament pour le traitement des maladies gastro-intestinales et des maladies de l'oesophage qui sont provoquées par une hypersécrétion d'acides biliaires.

21. Médicament, caractérisé par une teneur efficace en un composé 1 selon la revendication 1.
